# EUROPEAN PATENT APPLICATION

(11) **EP 4 603 102 A1**
(43) Date of publication of application: **20.08.2025**
(21) Application number: 23877413.7
(22) Date of filing: 15.06.2023
(51) Int. Cl.: A61K 35/48, A61K 38/39, A61K 38/17, A61P 15/02, C09D 11/00, A61L 27/36, C12N 5/071, B01L 3/00

(54) **COMPOSITION FOR ENDOMETRIAL REGENERATION CONTAINING DECELLULARIZED EXTRACELLULAR MATRIX DERIVED FROM UTERUS AND MANUFACTURING METHOD THEREFOR**

(30) Priority: 13.10.2022 KR 20220131178
(71) Applicant: POSTECH Research and Business Development Foundation, Pohang-si, Gyeongsangbuk-do 37673 (KR); Cha University Industry-Academic Cooperation Foundation, Pocheon-si, Gyeonggi-do 11160 (KR)
(72) Inventor: CHO, Dong Woo, Pohang-si Gyeongsangbuk-do 37673 (KR); JANG, Jin Ah, Pohang-si Gyeongsangbuk-do 37673 (KR); SEN, Tugce, Pohang-si Gyeongsangbuk-do 37673 (KR); AHN, Jung Ho, Seongnam-si Gyeonggi-do 13557 (KR); LEE, Dan Bi, Gwangju-si Gyeonggi-do 12784 (KR); BAE, Mi Hyeon, Pohang-si Gyeongsangbuk-do 37673 (KR); KANG, Youn Jung, Yongin-si Gyeonggi-do 16990 (KR)
(74) Representative: Stöckeler, Ferdinand
(86) International application number: PCT/KR2023/008298
(87) International publication number: WO 2024/080482

(57) **Abstract**

The present invention relates to a composition for endometrial regeneration containing a uterus-derived decellularized extracellular matrix (UdECM) and a manufacturing method therefor. Characterized by containing uterus-derived decellularized extracellular matrix (UdECM), the composition can exhibit the effect of enhancing the regeneration, receptivity, and implantation capabilities of the endometrium.

## Description

### [Technical Field]

The present invention relates to a uterine tissue-specific extracellular matrix that can improve the regeneration, receptivity, and implantation abilities of the endometrial (endometrium), and particularly to a composition for endometrial regeneration, including a uterus-derived decellularized extracellular matrix (UdECM), and a manufacturing method therefor.

### [Background Arts of the Invention]

The uterus is one of the main reproductive organs of women and is composed of several tissue layers (endometrium, myometrium, and serosa).

Approximately 10% of cases of female infertility are related to problems with the health of the uterus. Healthy endometrial tissue is very important for successful embryo implantation and maintaining pregnancy. In particular, the thickness of the endometrium is one of the main characteristics that have been used as an indicator of endometrial receptivity. Many studies have reported that a sufficient thickness of the endometrium is essential for successful implantation and pregnancy. In other words, if the thickness of the endometrium is too thin (less than 7 mm), it becomes difficult to maintain pregnancy.

For this reason, various treatment methods are being studied to improve the thickness of the endometrium, and despite various treatment strategies including hormone therapy, intrauterine injection of growth factors, and the use of vasoactive substances, it is still difficult to see clinically significant effects, and the regeneration of the endometrium remains a challenging aspect of female infertility treatment.

To overcome these limitations, transplantation of individual extracellular matrix (ECM) components from specific tissues or biomaterials based on collagen or hyaluronic acid has been used to regenerate the endometrium. However, such single ECM-based biomaterials have the limitation of being unable to mimic tissue-specific extracellular matrices that support tissue-specific microenvironments and cell-to-cell interactions based on unique combinations of various proteins.

### [Related Technical Documents]

### [Patent Documents]

(Patent Document 1) Korean open-laid patent No.10-2021-0018639 (Laid open Date: Feb. 18, 2021)

### [Summary of the Invention]

### [Technical Subject]

The present invention aims to provide a composition for endometrial regeneration that can improve the regeneration, receptivity, and implantation abilities of the endometrium to solve the above-mentioned problems.

The present invention also aims to provide a composition for endometrial regeneration that contains collagens, glycoproteins, and proteoglycans, increases the expression of the receptors of the above hormones when treated with steroid hormones, and has the functions of endometrial regeneration and promotion of angiogenesis.

In addition, the present invention aims to further improve the responsiveness to steroid hormones and to further enhance the receptivity of the endometrium.

### [Technical Solution]

One exemplary embodiment of the present invention to achieve the above objectives may be a composition for endometrial regeneration comprising uterus-derived decellularized extracellular matrix (UdECM).

Preferably, but not necessarily, the uterus may be a uterus derived from a pig.

Preferably, but not necessarily, the uterus-derived decellularized extracellular matrix may be a whole-uterine decellularized extracellular matrix (Whole-UdECM) derived from the whole uterus, including the endometrium, myometrium, and perimetrium.

Preferably, but not necessarily, the uterus-derived decellularized extracellular matrix may be a decellularized extracellular matrix (Endo-UdECM) derived from the endometrium.

Preferably, but not necessarily, the uterus-derived decellularized extracellular matrix (UdECM) may be an Endo-UdECM derived from endometrium isolated from the whole uterus including endometrium, myometrium and perimetrium.

Preferably, but not necessarily, the uterus-derived decellularized extracellular matrix may be Endo-UdECM derived from endometrium separated from uterine tissue treated with surfactant after treating the surfactant on the whole uterus including endometrium, myometrium and perimetrium.

Preferably, but not necessarily, a composition for endometrial regeneration comprising whole-UdECM derived from the uterine tissue (Whole Uterus) may be preferably intended for patients with uterine synechiae.

Preferably, but not necessarily, the composition for endometrial regeneration according to the present invention may include collagen, glycoprotein, and proteoglycan components.

Preferably, but not necessarily, the composition for endometrial regeneration according to the present invention can increase the expression of the receptor of hormone when treated with a steroid hormone and has the function of promoting regeneration of the endometrium and angiogenesis.

Another exemplary embodiment of the present invention to achieve the above objectives may be a manufacturing method of composition for endometrial regeneration, comprising the steps of:
preparing the whole uterus, which includes the endometrium and myometrium;
treating the prepared uterine tissue with a first surfactant;
separating the endometrium from the uterine tissue treated with the first surfactant; and
treating the separated endometrium with a second surfactant.

Preferably, but not necessarily, the first surfactant may be sodium dodecyl sulfate (SDS).

Preferably, but not necessarily, the second surfactant may be Triton X-100.

Still another exemplary embodiment of the present invention to achieve the above objectives may be a bioink composition comprising uterus-derived decellularized extracellular matrix (UdECM).

Still further exemplary embodiment of the present invention to achieve the above objectives may be a composition for culturing uterus organoids, comprising uterus-derived decellularized extracellular matrix (UdECM).

Still further exemplary embodiment of the present invention to achieve the above objectives may be a composition for producing an organ on a chip, comprising uterus-derived decellularized extracellular matrix (UdECM).

The specific details of other embodiments are included in the detailed description and drawings.

### [Advantageous Effects]

The composition for endometrial regeneration according to the present invention is characterized by the inclusion of uterus-derived decellularized extracellular matrix (UdECM), which has the effect of improving the regeneration, receptivity, and implantation capacity of the endometrium.

The composition for endometrial regeneration according to the present invention includes collagens, glycoproteins, and proteoglycans, increases the expression of the receptors of the above hormones upon treatment with steroid hormones, and has the functions of promoting regeneration of the endometrium and angiogenesis.

In addition, the composition for endometrial regeneration according to the present invention is more excellently responsive to steroid hormones and has an effect of increasing the water solubility of the endometrium.

### [Brief Description of Drawings]

FIG. 1 shows the proportion of matrisome proteins obtained by performing proteomic analysis of Endo-UdECM and Whole-UdECM according to an exemplary embodiment of the present invention.
FIG. 2 is a heatmap showing the specific components of the concentrated proteoglycans, collagens, and glycoproteins contained in Endo-UdECM and Whole-UdECM according to an exemplary embodiment of the present invention.
FIG. 3 is a schematic diagram showing the time progression for each hormone treatment in the in vitro evaluation of steroid hormone responsiveness for Endo-UdECM and Whole-UdECM according to an exemplary embodiment of the present invention.

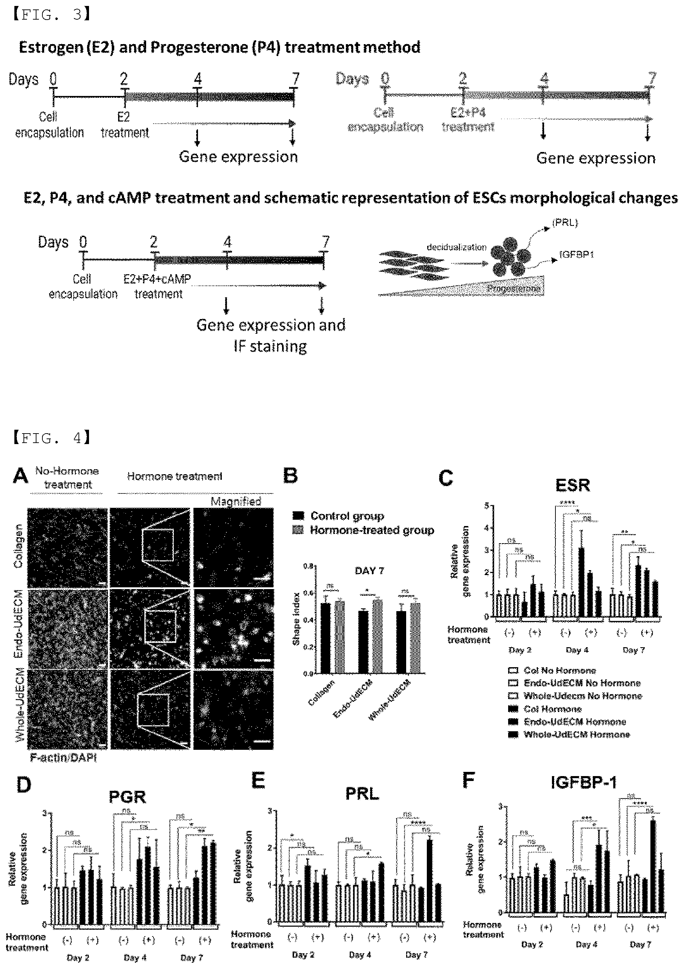

FIG. 4 is the result of the evaluation of the steroid hormone responsiveness of FIG. 3, and shows the ovarian steroid hormones (estrogen (E2) and progesterone (P4)) morphological differentiation of human endometrial stromal cells responding to E2 (A in FIG. 4), shape index calculated from the round shape of the embryonic stem cell form that changes according to the steroid hormone (B in FIG. 4), q-PCR analysis showing estrogen receptor expression (ESR) of E2 and P4-encapsulated ESCs (C in FIG. 4), respectively, and qRT-PCR analysis results for the expression of progesterone receptor (PGR) in E2- and P4-encapsulated ESC (D in FIG. 4) and the RT-PCR analysis results for the expression of decidualization markers (PRL and IGFBP-1) in E2+P4+cAMP-responsive encapsulated ESC (E and F in FIG. 4).

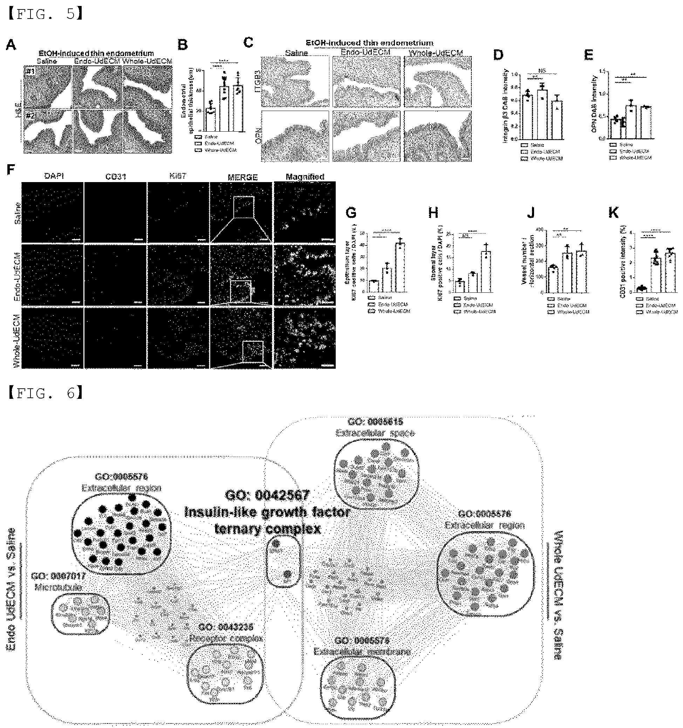

FIG. 5 verifies the injection effect by injecting Endo-UdECM and Whole-UdECM into a thin endometrial model according to an exemplary embodiment of the present invention. H&E stained image (A in FIG.5) and endometrial thickness (B in FIG. 5) after treatment in a thin endometrial model, immunohistochemical staining of ITGβ3 and OPN (C in FIG. 5) in a thin endometrium treated with saline or UdECM (endo or whole), visualized protein expression by DAB intensity and quantification of ITGβ3, OPN staining results (D and E in FIG. 5), co-immunofluorescence staining of CD31 (red) and Ki67 (green) in a thin endometrial model treated with UdECM (endo or whole) (F in FIG. 5), and results of the comparison of Ki67-positive cells (G and H in Fig. 5), total number of blood vessels (J in FIG. 5), and CD31-positive intensity (K in FIG. 5) in each group.
FIG. 6 is a result of gene ontology analysis of thin endometrial models treated with Endo-UdECM and Whole-UdECM according to an exemplary embodiment of the present invention.

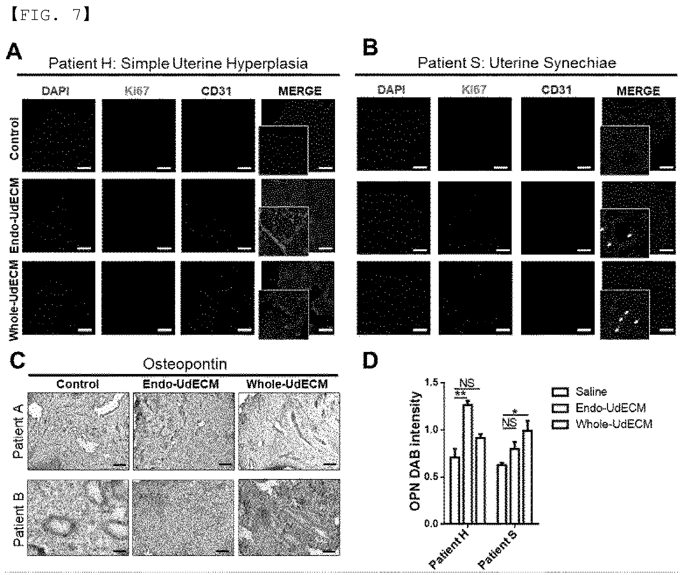

FIG. 7 is the result of in vitro efficacy verification of Endo-UdECM and Whole-UdECM on human endometrial tissue according to an exemplary embodiment of the present invention, and shows co-immunofluorescence staining of Ki67 (green) and CD31 (red) in human endometrial tissue {patient H sample (A) and patient S sample (B)} cultured with Endo-UdECM or Whole-UdECM-containing medium compared to control medium (A, B in FIG. 7), representative images of immunostaining for OPN in human endometrial tissue cultured with Endo-UdECM or Whole-UdECM-containing medium compared to control medium (C in FIG. 7) and a comparison of OPN expression in each group (D in FIG. 7).

### [Detailed Description]

The invention is subject to various modifications and can have many different embodiments, some of which are illustrated in the drawings and described in the accompanying detailed description. However, this is not intended to limit the invention to any particular embodiment, and should be understood to include all modifications, equivalents or substitutions that fall within the scope of the present idea and technology. In describing the invention, where it is believed that a detailed description of the relevant prior art would obscure the gist of the invention, such detailed description is omitted.

The terminology used in this application is intended to describe particular embodiments only and is not intended to limit the invention. Singular expressions include the plural unless the context clearly indicates otherwise. In this application, the terms such as 'comprising' or 'including' are used to indicate the presence of the features, numbers, steps, operations, components, parts, or combinations thereof described in the specification and are not intended to preclude the possibility of the presence or addition of one or more other features, numbers, steps, operations, components, parts, or combinations thereof.

Terms such as first, second, and the like may be used to describe various components, but the components are not to be limited by such terms. They are used only for the purpose of distinguishing one component from another.

One exemplary embodiment of the present invention is a composition for endometrial regeneration comprising uterus-derived decellularized extracellular matrix (UdECM).

The present invention relates to a uterine (uterus) tissue-specific extracellular matrix that can improve the regeneration, receptivity, and implantation abilities of the endometrium, and in particular, a composition for endometrial regeneration, including a uterus-derived decellularized extracellular matrix (UdECM), and a manufacturing method therefor.

In this specification, 'uterine-derived decellularized extracellular matrix (UdECM)' refers to the extracellular matrix that can be obtained by decellularizing the tissues of the uterus of humans or animals.

Among them, it is possible that the above-mentioned uterus-derived extra-cellular matrix (UdECM) is pig-uterus derived extra-cellular matrix (P-UdECM). There are no particular restrictions on the type of pig or the part of the uterus tissue, and the method of decellularization can use various methods known in this field of technology. For example, although the tissue is different, the pig uterus tissue can be decellularized using the method already developed by the inventors (Korean Patent Registration No. 10-2282073), and It is possible to obtain pig uterus-derived decellularized extracellular matrix (P-UdECM) by freeze-drying the above-mentioned decellularized tissue, grinding it, adding protein-digesting enzymes and acid to the ground tissue, and dissolving it while stirring, and then adding a basic solution to the dissolved solution to adjust the pH.

UdECM actually contains tissue-specific extracellular matrix components, which can provide the physical, mechanical, and biochemical environment of the corresponding tissue and is highly efficient in promoting differentiation into uterine tissue cells and tissue-specific functionality.

The above decellularized extracellular matrix refers to a natural support for cell growth manufactured through the decellularization of tissues found in mammals and multicellular organisms. The extracellular matrix mentioned above includes collagen, elastins, laminins, glycosaminoglycans, proteoglycans, antimicrobials, and chemoattractants. It may be a mixture of structural and non-structural biomolecules, including but not limited to growth factors. The extracellular matrix may be in various forms in mammals and may contain approximately 90% collagen. Extracellular matrix derived from various tissues may differ in their overall structure and composition due to the unique roles required for each tissue.

The composition for endometrial regeneration according to the present invention is characterized by the inclusion of such an endometrial-derived decellularized extracellular matrix (UdECM).

Previously, transplantation of biomaterials based on individual extracellular matrix (ECM) components of a specific tissue, collagen or hyaluronic acid, has been used for endometrial regeneration. However, such single ECM-based biomaterials have the limitation of being unable to mimic tissue-specific extracellular matrix that support tissue-specific microenvironments and intercellular interactions based on unique combinations of various proteins.

The inventors focused on the fact that using uterus-derived decellularized extracellular matrix would be most suitable for effective regeneration of the endometrium, and developed a method for producing a whole-uterus decellularized extracellular matrix (Whole-dECM) and additionally isolated a specific tissue layer of the uterus to produce Endometrium Uterus Decellularized Extracellular matrix (Endo-UdECM). Then, the protein analysis, responsiveness to steroid hormones, and in vivo/in vitro analysis of therapeutic efficacy were performed for each UdECM manufactured above to verify the clinical efficacy and tissue-specific properties of each UdECM, and the invention was completed.

According to the present invention, the composition for endometrial regeneration is characterized by uterus-derived decellularized extracellular matrix (UdECM), as shown in the following examples and experiments, and has the effect of improving the regeneration, receptivity, and implantation capacity of the endometrium.

As an exemplary embodiment of the present invention, the decellularized extracellular matrix derived from the uterus may be a whole-uterine decellularized extracellular matrix (Whole-UdECM) derived from the whole uterus, including the endometrium, myometrium, and perimetrium.

Generally, the uterine tissue consists of three membranes. Therefore, in a particular part of the uterus, the uterine tissue may include the endometrium, the myometrium, and the serosa. The present invention is possible to be a composition including an extracellular matrix that has been decellularized from the uterine tissue including the endometrium, the myometrium, and the serosa.

The composition for endometrial regeneration according to the present invention includes collagens, glycoproteins, and proteoglycans, increases the expression of the receptors of the above hormones upon treatment with steroid hormones, and has the functions of regeneration of the endometrium and promotion of angiogenesis.

As an embodiment of the present invention, the uterus-derived decellularized extracellular matrix may be a decellularized extracellular matrix (Endo-UdECM) derived from the endometrium.

The Endo-UdECM derived from the endometrium may be an extracellular matrix that has been separated from the endometrium from the uterine tissue of a human or animal and then subjected to a decellularization process from the beginning, or it may be an extracellular matrix that has been subjected to a decellularization process after the endometrium has been separated from the uterine tissue in the middle of the process of decellularizing the uterine tissue.

As an example, the decellularized extracellular matrix of the uterus may be a decellularized extracellular matrix (Endo-UdECM) derived from the endometrium (Endometrium) isolated from the whole uterus, including the endometrium, myometrium, and serosa.

Thus, a composition comprising an Endo-UdECM derived from the endometrium is more excellently responsive to steroid hormones, more receptive to the endometrium, and more effective, especially in patients with endometrial hyperplasia.

As an embodiment of the present invention, it is possible that the decellularized extracellular matrix derived from the endometrium is a decellularized extracellular matrix derived from the endometrium (Endo-UdECM) separated from the endometrium (Endometrium) treated with a surfactant and then treated with a surfactant in the whole uterus, including the endometrium, myometrium, and the outer membrane of the uterus.

In other words, the Endo-UdECM derived from the endometrium is an extracellular matrix that is derived from the endometrium after the endometrium is separated from the uterine tissue during the process of decellularizing the uterine tissue and then undergoes a decellularization process, where the time to separate the endometrium is preferably during the decellularization process, specifically after treatment with a surfactant, from the uterine tissue to separate the endometrium.

The inventors studied the protocol for decellularizing uterine tissue and endometrium based on a combination of surfactants including sodium dodecyl sulfate (SDS) and Triton X-100. To obtain Endo-UdECM separately, the endometrial layer swollen after SDS treatment was separated from the uterine muscle layer, and then afterwards, it was found that the most effective way was to perform the decellularization process. In other words, after treating the uterine tissue with SDS, the endometrium swells in the myometrium, and because of this, separating the endometrium after SDS treatment allows the most effective separation of the endometrium without losing tissue, and the decellularized extracellular matrix of the endometrium can be obtained.

As an exemplary embodiment of the present invention, a composition for endometrial regeneration comprising whole-UdECM derived from the above-mentioned uterine tissue (Whole Uterus) is preferably intended for patients with uterine synechiae.

As shown in the experimental examples described below, the inventors tested the in vitro efficacy of Endo-UdECM and Whole-UdECM on human endometrial tissue, and as a result, Whole-UdECM derived from uterine tissue showed better expression of OPN in the tissue of patients with uterine synechiae.

As an exemplary embodiment of the present invention, a composition for endometrial regeneration comprising an Endo-UdECM derived from the endometrium is preferably intended for patients with uterine hyperplasia.

As shown in the experimental examples described below, the inventors tested the in vitro efficacy of Endo-UdECM and Whole-UdECM on human endometrial tissue, and as a result, Endo-UdECM derived from the endometrium showed better expression of OPN (Osteopontin) in the tissues of patients with uterine hyperplasia.

The composition for endometrial regeneration according to the present invention, which is described above, basically includes uterine-derived decellularized extracellular matrix (UdECM), and it is also possible to additionally include other biomaterials such as collagen and/or hyaluronic acid.

These biomaterials may be encapsulated in the empty spaces of the decellularized extracellular matrix (UdECM) derived from the decellularized cells of the uterus. Therefore, the UdECM derived from the decellularized cells of the uterus, which is encapsulated with biomaterials like this, does not deteriorate over time, and it can have excellent endometrial regeneration function even when cultured for a long period of time.

One exemplary embodiment of the present invention may further include a hydrogel.

In other words, the composition for endometrial regeneration according to the present invention basically includes a uterus-derived decellularised extracellular matrix (UdECM), and it is also possible to include a hydrogel additionally selected thereto.

The hydrogel mentioned above may include various types of hydrogels known in this technical field without being particularly limited. For example, the hydrogel may be a natural hydrogel such as collagen or agarose, or it may be a synthetic hydrogel consisting of PLGA, etc.

In the present invention, the hydrogel has the function of supporting or holding the uterine-derived decellularized extracellular matrix (UdECM) and/or other biomaterials, thereby further enhancing the efficacy of endometrial regeneration of the said extracellular matrix.

Accordingly, an embodiment of the present invention may be a hydrogel mixed with uterine-derived decellularized extracellular matrix (UdECM) and/or other biomaterials according to the present invention. Furthermore, the above-mentioned decellularized extracellular matrix (UdECM) and/or other biomaterials may be mixed and encapsulated (encapsulation) in the hydrogel. In this case, the above-mentioned extracellular matrix (UdECM) and/or other biomaterials are mixed and/or fused at a short distance, which has the effect of maximizing the responsiveness and acceptability to steroid hormones.

Another exemplary embodiment of the present invention may comprise the steps of: preparing whole uterus, which includes endometrium and myometrium; treating the prepared uterus with a first surfactant; separating endometrium from the uterus treated with the first surfactant; and treating the separated endometrium with a second surfactant.

The above-mentioned whole uterus may include the endometrium and the myometrium, and may optionally include the extra-uterine membrane. It is possible to obtain uterine tissue from the uterus of mammals, including humans and animals.

The prepared uterine tissue can then be cut into suitable sizes and further processed by washing with water or distilled water.

The uterine tissue can then be treated with a first surfactant. The first surfactant is not particularly limited and can be any of a variety of surfactants known in the art. For example, sodium dodecyl sulfate (SDS) or Triton X-100 may be used, and preferably SDS is suitable.

Then, the present invention comprises a step of separating the endometrium from the uterine tissue treated with the first surfactant. In other words, the separation of the endometrium is performed after treating the uterine tissue with the first surfactant. As mentioned above, after treating the uterine tissue with a surfactant, the endometrium swells in the myometrium, so separating the endometrium after treatment with a surfactant is the most effective way to separate the endometrium without losing tissue.

This may then include a further process of washing the separated endometrium with water or distilled water.

This may include a step of treating the separated endometrium with a second surfactant. The second surfactant may be any surfactant known in the art, without being particularly limited. For example, sodium dodecyl sulfate (SDS) or Triton X-100 may be used, preferably Triton X-100.

Afterwards, the endometrium treated with the second surfactant may further include a process of washing with water or distilled water.

It is also possible to add protein-digesting enzymes and/or acids to the endometrium, stir and dissolve, and add a basic solution to the dissolved solution to adjust the pH.

Another exemplary embodiment of the present invention is a bioink composition comprising uterus-derived decellularized extracellular matrix (UdECM).

In other words, a composition comprising uterine-derived decellularized extracellular matrix (UdECM) according to the present invention has the effect of improving the regeneration, receptivity, and implantation capacity of the endometrium, and such a composition can also be used as a three-dimensional bioink for manufacturing uterine tissue models.

According to the present invention, a culture environment similar to the human uterus can be established based on the decellularized extracellular matrix derived from the uterus, and this has the effect of most closely mimicking the human uterus environment in vitro, unlike other uterine tissue decellularization compositions reported to date.

The bio-ink composition according to the present invention may further include various biodegradable materials, bioactive materials, hydrogels, crosslinkers, etc. known in the art.

Still another embodiment of the present invention is a composition for culturing uterus organoids, comprising uterus-derived decellularized extracellular matrix (UdECM).

In other words, a composition comprising uterus-derived decellularized extracellular matrix (UdECM) according to the present invention has the effect of improving the regeneration, receptivity, and implantation capacity of the endometrium, and such a composition may also be used as a composition for culturing uterine organoids.

The above 'organoid' refers to a miniature organ produced in the form of an artificial organ by culturing cells derived from tissues or pluripotent stem cells in a 3D form. The above organoids are three-dimensional tissue analogs that include organ-specific cells that arise from stem cells and self-organise (or self-pattern) in a manner similar to their in vivo state, and can develop into specific tissues by limited factor (e.g. growth factor) patterning.

The endometrial organoids cultured from the composition according to the present invention have excellent regeneration, receptivity, and implantation abilities of the endometrium, and can be developed in a form that is structurally similar to the actual endometrium.

Still another embodiment of the present invention is a composition for producing an organ on a chip, which includes uterus-derived decellularized extracellular matrix (UdECM).

In other words, a composition comprising uterus-derived decellularized extracellular matrix (UdECM) according to the present invention has the effect of improving the regeneration, receptivity, and implantation capacity of the endometrium, and such a composition may also be used as a composition for the manufacturing of organ chips and/or bioprinted organs. The term 'bioprinted organ' refers to an organ formed by 3D or bioprinting methods.

The existing matrigel-based culture systems are extracts derived from animal cancer tissue, which vary greatly between batches, do not mimic the actual environment of the uterus, and have insufficient efficiency for differentiation and development into uterine organoids. In contrast, the composition of the present invention can create an environment similar to endometrial tissue, making it suitable for uterine organ chips and/or bioprinted organ culture.

Still another embodiment of the present invention is a method for culturing the endometrium, which includes the step of culturing the endometrium in the endometrial regeneration composition described above.

The specific method or process for culturing the endometrium in the endometrial regeneration composition described above is not particularly limited. The above-mentioned culture refers to the process of maintaining and growing the endometrium under suitable conditions, and suitable conditions may refer to, for example, the temperature at which the endometrium is maintained, nutrient availability, atmospheric CO₂ content, and cell density.

The present invention can be better understood by the following embodiments, which are for illustrative purposes only and do not limit the scope of protection as defined by the attached patent claims.

### Exemplary Embodiment 1: Manufacture of uterus-derived decellularized extracellular matrix

Using porcine (pig) uterus, inventors produced whole uterine decellularized extracellular matrix (Whole-UdECM) and endometrial decellularized extracellular matrix (Endo-UdECM).

Until now, no study has shown the differences in the composition and function of the entire uterine tissue and endometrium, a specific part of the uterine tissue.

The inventors studied the protocol for decellularization of uterine tissue and endometrium based on a combination of surfactants including sodium dodecyl sulphate (SDS) and Triton X-100.

The specific decellularization process is shown in Table 1 below.

**[Table 1]**

| Porcine uterus-derived Whole-UdECM and Endo-UdECM de cellularization process | | | | |
|---|---|---|---|---|
| **Treatment process** | | **methods** | | |
| | | **Tempera ture (°C)** | **Durat ion (h)** | **shaking speed (rpm)** |
| 1 | Separate the outer layer of tissue from the pig uterus. | N/A | N/A | N/A |
| 2 | Cut the separated uterus into pieces of about 20 mm × 25 mm. | RT | N/A | N/A |
| 3 | Double-distilled water (DDW) is used for cleaning. | RT | 2h | 110 |
| 4 | Treat with 1% SDS at DDW (change solution after the first hour, then every 12 hours). | RT | 72h | 110 |
| 5-1 | Endo-UdECM: The endometrial layer was isolated from the myometrium to obtain dECM derived from the endometrium. | RT | N/A | N/A |
| 5-2 | Whole-UdECM: No stratification of the uterus-derived dECM was performed. | | | |
| 6 | Clean with double distilled water (DDW). | RT | 48 | 110 |
| 7 | Treat DDW with 0.5% Triton X-100. | RT | 18 | 110 |
| 8 | Clean with double distilled water (DDW). | RT | 30 | 110 |
| 9 | Treatment with 30 U/ml DNase in 1X PBS containing 1.3 mM MgCl₂ and 2 mM CaCl₂ | 37 | 24 | 100 |
| 10 | Clean with double distilled water | RT | 24 | 110 |
| | (DDW). | | | |
| 11 | 0.1% of the peracetic acid is treated. | RT | 2 | 110 |
| 12 | Wash with sterile 1X PBS. | RT | 24 | 110 |
| 13 | Freeze-dry. | N/A | 48 | N/A |

The overall process of obtaining whole-UdECM, which is derived from the whole uterus, and Endo-UdECM, which is derived from the endometrium, is similar. However, in order to obtain Endo-UdECM separately, the endometrial layer that swelled after the above-mentioned SDS treatment was separated from the myometrial layer, and then the de-cellularization process was carried out.

In other words, the de-cellularization process of Endo-UdECM and Whole-UdECM was the same up to No. 4 in Table 1 above. The difference between Endo-UdECM and Whole-UdECM is the separation of the two in No.5. After SDS treatment of the uterine tissue, the endometrium swells in the myometrium. This is why it was found that separating the endometrium after SDS treatment is advantageous for proceeding without tissue loss. After that, only the swollen endometrial tissue was removed separately, and the decellularization process was carried out by separating it from the process N0. 6.

Through this decellularization process, different endometrial regeneration compositions containing Endo-UdECM and Whole-UdECM were prepared.

### Experimental example 1: Comparison of the components of the extracellular matrix of decellularized cells

Proteomics analysis was performed on Whole-UdECM and Endo-UdECM manufactured according to the above embodiment 1.

The protein lysate was prepared as follows: 10 mg of freeze-dried Whole-UdECM and Endo-UdECM were prepared, respectively, and then 10 µg of Lys-C/Trypsin and 700 µL of 50 mM TEAB buffer were added, and incubated at 37°C for 16 hours. Afterwards, desalting was performed using SEK-PAC C18, followed by freeze-drying. This sample was then dissolved in 100 ul of 0.1% formic acid and liquid chromatography-mass spectrometry (LC-MS) was performed.

The proteins detected through LC-MS analysis were quantified and identified using Sequest HT in proteome discoverer 2.4 (Thermo) software. Each identified protein was classified using the UniprotKB database, and the matrisome analysis was performed using the Human Matrisome Reference (Atlas).

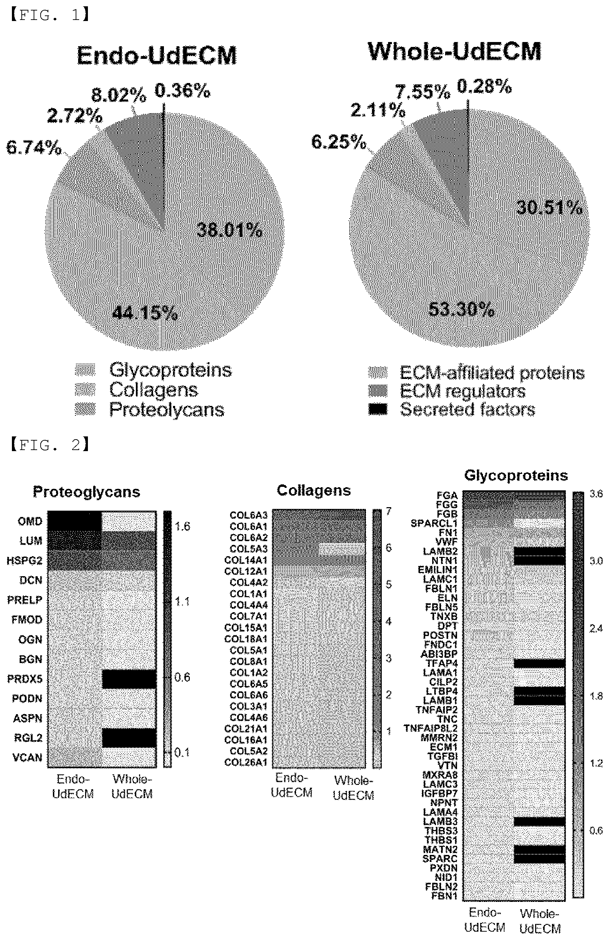

FIG. 1 shows the proportion of matrisome proteins obtained by performing a proteomic analysis of Endo-UdECM and Whole-UdECM according to one embodiment of the present invention.

The analysis showed that Endo-UdECM contains a greater amount of glycoproteins than Whole-UdECM in terms of the matrisome, while it contains less collagen (see FIG. 1).

FIG. 2 is a heatmap showing the specific components of concentrated proteoglycans, collagens, and glycoproteins contained in Endo-UdECM and Whole-UdECM according to one embodiment of the present invention.

When the protein composition was examined in more detail, both Whole-UdECM and Endo-UdECM were found to be most abundant in the collagen group, with COL type VI, and in the glycoprotein group, fibrinogen (FGA, FGB, FGG) was found to be the most abundant (see FIG. 2). Col type VI is an important component of human endometrial formation and is a protein that is highly expressed in the endometrium during the menstrual cycle before embryo implantation. Fibrinogen is also a protein that plays an important role in the vascular remodeling of the mother.

These results showed that the composition comprising Endo-UdECM and Whole-UdECM according to the present invention can provide a complex microenvironment for reproducing the function of the endometrium.

### Experimental example 2: In vitro evaluation of Endo-UdECM and Whole-UdECM on steroid hormone reactivity

The response of human endometrial stromal cells (hESCs) to steroid hormones is important for the receptivity of the endometrium and maintaining pregnancy.

Therefore, to confirm that the function of such cells can be reproduced in the composition according to the present invention, we verified the responsiveness to steroid hormones after enclosing hESCs in Whole-UdECM and Endo-UdECM prepared according to Example 1 and Collagen hydrogel as a comparison.

First, three experimental groups were created by encapsulating hESCs at a concentration of 50,000 cells/ml in 10 mg/ml of Endo-UdECM, Whole-UdECM, and collagen hydrogel, respectively.

The treatment of steroid hormones was carried out in three ways. In other words, three types of steroid hormones were administered to induce decidualization, which corresponds to the first seven days of the menstrual cycle.
1) After 2 days of cell encapsulation in hydrogel, 10 nM E2 (β-Estradiol, Sigma-Aldrich, E8875) was administered to each experimental group to observe the expression of estrogen and progesterone receptors, respectively.
2) After 2 days of cell encapsulation in hydrogel, each experimental group was treated with 10 nM E2 + 1 uM P4 (progesterone, Sigma-Aldrich, P8783) to observe the expression of estrogen and progesterone receptors, respectively.
3) In addition, each experimental group was treated with a combination of 10 nM E2 + 1 uM P4 + 1 uM cAMP (8-Bromoadenosine 3',5'-cyclic monophosphate sodium salt, Sigma-Aldrich, B7880) for 7 days to induce desensitization.

The time progression for each hormone treatment is shown in FIG. 3. FIG. 3 is a schematic diagram showing the time progression for each hormone treatment in the in vitro evaluation of steroid hormone responsiveness of Endo-UdECM and Whole-UdECM according to one embodiment of the present invention. Furthermore, the results are shown in FIG. 4.

FIG. 4 is the result of the evaluation of steroid hormone responsiveness in FIG. 3, showing that ovarian steroid hormones (estrogen (E2) and progesterone (P4)) morphological differentiation of human endometrial stromal cells responding to E2 (A in FIG. 4), shape index calculated from the round shape of the embryonic stem cell form that changes according to the steroid hormone (B in FIG. 4), and q-PCR analysis showing estrogen receptor expression (ESR) of E2 and P4-encapsulated ESCs (C in FIG. 4), respectively. The qRT-PCR analysis results for the expression of progesterone receptor (PGR) in E2- and P4-encapsulated ESC (D in Fig. 4) and the RT-PCR analysis results for the expression of decidualization markers (PRL and IGFBP-1) in E2+P4+cAMP-responsive encapsulated ESC (E, F in FIG. 4) are shown.

Seven days after hormone treatment, hESCs cultured on Endo-UdECM showed a significant change in the spindle-shaped cell morphology to a round shape as an indicator of improved water solubility of the endometrium (A, B in FIG. 4). In addition, we observed that the expression of the reproductive hormone receptors estrogen (ESR) and progesterone (PGR) was significantly higher in the Endo-UdECM group and Whole-UdECM group (C, D in FIG. 4). In addition, we found that the expression of PRL and IGFBP-1, markers of hormone receptivity, was significantly increased in the Endo-UdECM group (E, F in FIG. 4).

### Experimental Example 3: The effect of direct intrauterine injection of Endo-UdECM and Whole-UdECM in a thin endometrial model

The animal model (disease model) with a thin endometrium was created by injecting 95% ethanol (EtOH) into the uterus of a female mouse in estrus. Specifically, the disease model used a 31G insulin syringe to inject 40 ul of 95% ethanol into one wall of the uterus, while the normal control group injected 40 ul of saline into the opposite wall of the uterus using the same method. After four days of estrus (one estrus cycle) following ethanol and saline injection, the thin endometrial model was divided into the Endo-UdECM injection group and the Whold-UdECM injection group, and each UdECM was injected into the uterus to verify the therapeutic effect. In addition, the saline injection group was used as a control group.

The results are shown in FIG. 5. FIG. 5 verifies the effect of injection by injecting Endo-UdECM and Whole-UdECM according to one embodiment of the present invention into a thin endometrial model. H&E stained image (A in FIG. 5) and endometrial thickness (B in FIG. 5) after treatment in a thin endometrial model, immunohistochemical staining of ITGβ3 and OPN (C in FIG. 5) in a thin endometrium treated with saline or UdECM (Endo or Whole), visualized protein expression (D and E in FIG. 5) by DAB intensity and quantification of ITGβ3 and OPN staining results, co-immunofluorescence staining of CD31 (red) and Ki67 (green) in a thin endometrial model treated with UdECM (Endo or Whole) (F in FIG. 5), the results of the comparison of Ki67-positive cells (G and H in FIG. 5), total number of blood vessels (J in FIG. 5), and CD31-positive intensity (K in FIG. 5) in each group.

When Endo-UdECM and Whole UdECM were injected into a thin endometrial model, the thickness of the endometrium was significantly increased, unlike the saline injection group (FIG. 5A, B). Integrinβ3 and Osteopontin (OPN), markers of the receptivity of the endometrium, were significantly increased in the Endo-UdECM treatment group, while only OPN was significantly increased in the Whole-UdECM treatment group, and the level of Integrinβ3 did not change significantly (D, E in FIG. 5).

The main characteristics of endometrial pathology are ischemic pathology of the endometrium and proliferation of the damaged endometrium. Accordingly, in this experiment, we examined whether Endo-UdECM and Whole-UdECM can promote the formation of new blood vessels in the endometrium and normalize (promote) cell proliferation in the endometrium. First, the Whole-UdECM treatment group had a higher number of cells expressing Ki67, a cell proliferation marker, in both the epithelium and stromal layer compared to the control group (saline treatment group). In contrast, the Endo-UdECM-treated group showed an increase in the number of Ki67-positive cells limited to the epithelium, suggesting that it can induce tissue-specific cell proliferation (F, G, H in FIG. 5). In addition, the vascularization marker CD31 was significantly increased in both the Endo-UdECM and Whole-UdECM treatment groups, suggesting that both UdECMs can promote vascularization (F, J, K in FIG. 5).

### Experimental example 4: Investigation of the common therapeutic mechanisms of Endo-UdECM and Whole-UdECM

The regulatory mechanisms of UdECMs were investigated by analyzing the gene ontology of thin endometrial models treated with Endo-UdECM and Whole-UdECM, respectively.

FIG. 6 shows the results of the gene ontology analysis of thin endometrial models treated with Endo-UdECM and Whole-UdECM according to one embodiment of the present invention. Cell components GO terms classified by Endo UdECM-treated uterus, saline-treated uterus, and Whole UdECM-treated uterus. Analysis of the gene-gene interaction network of DEG in the saline-treated uterus showed a common GO term (0042567).

Each treatment condition of Endo-UdECM and Whole-UdECM expressed a common GO-Term for the insulin-like growth factor ternary complex, which included IGF1 and IGFBP3. In particular, the expression of IGF1 was significantly lower than that of saline treatment in all conditions of UdECM treatment, but the expression of IGFBP3 was conversely higher in the UdECM treatment conditions. Based on this, we assumed that the main mechanisms of the therapeutic efficacy of UdECM would be downregulation of IGF1 and upregulation of IGFBP3.

Accordingly, in this experimental example, it was assumed that UdECM+IGF1 injection would weaken the efficacy of UdECM, while IGFBP3 monotherapy would have a therapeutic efficacy similar to that of UdECM.

To prove this, IGF1 was administered at a concentration of 1 mg/ml with Endo-UdECM or Whole UdECM to a thin endometrial mouse model. In addition, to verify the efficacy of IGFBP3, IGFBP3 was administered alone at a concentration of 1 mg/ml, and the therapeutic efficacy was verified for each experimental group.

The results of the experiment showed that the mixed treatment group of UdECM+IGF1 showed a significant reduction in endometrial thickness and epithelial thickness compared to the UdECM treatment group alone, confirming that IGF1 reduces the therapeutic efficacy of UdECM. In addition, in the IGFBP3-treated group, the recovery of the endometrial thickness and the epithelial thickness of the endometrium were confirmed to be similar to those of the UdECM-only treated group, confirming that IGFBP3 plays a similar role to that of UdECM in terms of therapeutic efficacy.

### Experimental example 5: In vitro efficacy of Endo-UdECM and Whole-UdECM on human endometrial tissue

Endometrial samples were collected from women under the IRB approval of the CHA Medical Center (Approval No. 2020-10-007). Endometrial samples were sliced into 1-2 mm³ pieces and cultured in the following medium on cell culture chamber slides (SPL 30108). Medium composition: DMEM/F12 + FBS 20% (v/v) + L-glutamine 1% (v/v)

Afterwards, 0.1% Endo-UdECM or 0.1% Whole-UdECM was added to the medium to verify the in vitro efficacy of each UdECM, and 1 mol-1 of NaOH or HCl was added to adjust the pH to 6.0.

The efficacy of Endo-UdECM and Whole-UdECM was verified by comparing the expression of CD31, a marker of angiogenesis, and Ki67, a marker of cell proliferation.

FIG. 7 is the result of in vitro efficacy verification of Endo-UdECM and Whole-UdECM on human endometrial tissue according to one embodiment of the present invention. Human endometrial tissue {patient H sample (A) and patient S sample (B)} cultured in media containing Endo-UdECM or Whole-UdECM compared to control media is a co-immunofluorescence staining of Ki67 (green) and CD31 (red) (A and B in FIG. 7), representative images of immunostaining for OPN in human endometrial tissue cultured with Endo-UdECM or Whole-UdECM-containing medium compared to the control medium (C) and the comparison of OPN expression in each group (D).

In the case of patient H, who had endometrial hyperplasia, the expression of CD31 was increased in both UdECM treatment groups. In contrast, in patient S, who had uterine adhesions, we observed increased Ki67 expression in both UdECM treatment groups. In addition, the endometrial-specific soluble marker OPN was significantly increased in patient H under Endo-UdECM treatment and in patient S under Whole-UdECM treatment. This suggests that Endo-UdECM and Whole-UdECM have different effects on endometrial hyperplasia and uterine adhesions, respectively. These results suggest that considering the regulation of tissue-specific dECM composition for patient- and disease-specific personalized treatment is essential for successful endometrial regeneration and fertility improvement.

In the above, the applicant has described the preferred exemplary embodiments of the present invention, but these embodiments are only one embodiment of the technical idea of the present invention, and any modification or correction that embodies the technical idea of the present invention should be interpreted as falling within the scope of the present invention.

## Claims

1. A composition for endometrial regeneration containing uterus-derived decellularized extracellular matrix (UdECM).

2. The composition of claim 1, wherein the uterus is a uterus derived from a pig (porcine).

3. The composition of claim 1, wherein the uterus-derived decellularized extracellular matrix is a whole-uterine decellularized extracellular matrix (Whole-UdECM) derived from the whole uterus, including the endometrium, myometrium, and perimetrium.

4. The composition of claim 1, wherein the uterus-derived decellularized extracellular matrix is a decellularized extracellular matrix (Endo-UdECM) derived from the endometrium.

5. The composition of claim 1, wherein the uterus-derived decellularized extracellular matrix (UdECM) is an Endo-UdECM derived from endometrium isolated from the whole uterus including endometrium, myometrium and perimetrium.

6. The composition of claim 1, wherein the uterus-derived decellularized extracellular matrix is Endo-UdECM derived from endometrium separated from uterine tissue treated with surfactant after treating the surfactant on the whole uterus including endometrium, myometrium and perimetrium.

7. The composition of any one claim of claim 1 to 6, comprising collagen, glycoprotein, and proteoglycan components.

8. The composition of claim 7, wherein the composition increases the expression of the receptor of hormone when treated with a steroid hormone and has the function of promoting regeneration of the endometrium and angiogenesis.

9. A manufacturing method of composition for endometrial regeneration, comprising the steps of:
preparing the whole uterus, which includes the endometrium and myometrium;
treating the prepared uterine tissue with a first surfactant;
separating the endometrium from the uterine tissue treated with the first surfactant; and
treating the separated endometrium with a second surfactant.

10. The method of claim 9, wherein the first surfactant is sodium dodecyl sulfate (SDS).

11. The method of claim 9, wherein the second surfactant is Triton X-100.

12. A bioink composition comprising uterus-derived decellularized extracellular matrix (UdECM).

13. A composition for culturing uterus organoids, comprising uterus-derived decellularized extracellular matrix (UdECM).

14. A composition for producing an organ on a chip, comprising uterus-derived decellularized extracellular matrix (UdECM).
